# EUROPEAN PATENT APPLICATION

(11) **EP 4 640 154 A1**
(43) Date of publication of application: **29.10.2025**
(21) Application number: 24171914.5
(22) Date of filing: 23.04.2024
(51) Int. Cl.: A61B 5/389, A61B 5/397, A61B 5/00, G16H 50/20

(54) **COMPUTER-IMPLEMENTED METHOD, AND EMG DEVICE TO MEASURE ELECTRIC ACTIVITY OF A MUSCLE**

(71) Applicant: Koninklijke Philips N.V., 5656 AG Eindhoven (NL)
(72) Inventor: VAN GORP, Hans, Eindhoven (NL); DOS SANTOS DA FONSECA, Pedro Miguel Ferreira, Eindhoven (NL); VAN GILST, Merel Marietje, 5656AG Eindhoven (NL); OVEREEM, Sebastiaan, Eindhoven (NL); VAN SLOUN, Ruud Johannes Gerardus, Eindhoven (NL)
(74) Representative: Philips Intellectual Property & Standards

(57) **Abstract**

There is provided a computer-implemented method for estimating sleep stages. The method comprises receiving an electromyography (EMG) signal representative of electric activity of a muscle of the subject during the sleep session, and providing only the EMG signal as an input to a machine learning model. The method comprises estimating a sleep stage during the sleep session based on an output of the machine learning model. The machine learning model is trained by providing, as a first input, a reference EMG signal representative of electric activity of a muscle of a reference subject during a reference sleep session, and providing, as a second input, a reference sleep stage signal representative of sleep stages of the reference subject during the reference sleep session. The machine learning model is trained by using the first input and the second input to estimate sleep stages based on only an EMG signal.

## Description

### FIELD OF THE INVENTION

The invention relates to a computer-implemented method for training a machine learning model. Further, the invention relates to a computer-implemented method for estimating sleep stages of a subject during a sleep session. Further, the invention relates to a processing system configured to perform the computer-implemented method. Further, the invention relates to a computer-readable storage medium comprising instructions which, when executed by a processing system, cause the processing system to carry out the computer-implemented method. Further, the invention relates to an electromyography (EMG) device adapted to measure electric activity of a muscle of a subject during a sleep session.

### BACKGROUND OF THE INVENTION

Information about sleep stages during sleep provides important information about sleep disorders. The clinical gold standard to perform sleep staging is via polysomnography (PSG). A PSG is performed in a hospital or a sleep center. During the PSG, the overnight sleep measurements are taken by using a plurality of modalities, such as electroencephalography (EEG), electrooculography (EOG), and electromyography (EMG). Based on the outcome of the sleep measurements, a human technician visually inspects these signals generated by the modalities to score the sleep stages. Commonly, the different sleep stages are Wake, Rapid Eye Movement (REM) sleep and non-REM (N1, N2 and N3) sleep, and a scored on non-overlapping segments (so-called epochs) of 30 seconds.

Sleep stage information is used in diagnosing Sleep-disordered breathing (SDB) conditions, such as OSA and CSA. SDB conditions are becoming increasingly common, and are particularly prevalent in older people, people with a high body mass index, smokers, heavy drinkers and people with conditions such as coronary artery disease, hypertension and diabetes mellitus.

SDB conditions are often treated using positive airway pressure (PAP) therapy, in which pressurized air is provided to a subject to keep the subject's airways open. When first prescribing PAP therapy, a PAP titration study is carried out for the subject in order to determine a level of airway pressure to be provided to the subject during PAP therapy, as well as a suitable PAP therapy modality (e.g. continuous positive airway pressure, CPAP, bilevel positive airway pressure, BiPAP, or automatic positive airway pressure, APAP) and a suitable subject interface (e.g. a nasal pillow, an oronasal/full-face mask).

Sleep stage information is used in diagnosing REM behavior disorder, RBD. Healthy people have muscle atonia during REM sleep. However, a subject having RBD does not have muscle atonia during REM sleep. RBD is a strong predictor of the development of Parkinson's disease. By using sleep stage information, it is determined whether a subject is in REM sleep. During the PSG, muscle activity is measured during the REM sleep to determine whether muscle atonia is present. Based on the sleep stage information and the measured muscle activity, the diagnosis of RBD can be determined.

### SUMMARY OF THE INVENTION

Performing a PSG is costly and can cause subject discomfort. The subject is connected to a tangle of wires and has to spend a night sleeping in a dedicated sleep laboratory instead of at home. A PSG includes EEG measurements, which provide an important and accurate contribution to scoring the sleep stages. However, the EEG electrodes need to be placed on various locations on the scalp of the subject, interfering with the subject's hairline.

It is an objective of the invention to provide a method for estimating sleep stages of a subject with improved comfort for the subject.

According to a first specific aspect, there is provided a computer-implemented method for training a machine learning model. The method comprises providing, as a first input, a reference electromyography (EMG) signal representative of electric activity of a muscle of a reference subject during a reference sleep session. The method comprises providing, as a second input, a reference sleep stage signal representative of sleep stages of the reference subject during the reference sleep session. The method comprises using the first input and the second input to train the machine learning model to estimate sleep stages based on only an EMG signal.

The inventors have discovered that an EMG signal has sufficient information that allows for the estimation of sleep stages of the subject. By training the machine learning model based on the reference EMG signal and the reference sleep stage signals, the machine learning model is trained to estimate sleep stages of a subject based on only an EMG signal. No further signals than the EMG signal are needed by the trained machine learning model to estimate the sleep stages. As a result, the subject only needs to be connected to an EMG sensor, such as an EMG electrode pair. As no further signals are needed, there is no need for any other sensor to be connected to the subject. Also, the EMG sensor can be connected to the subject at a convenient location, such as on the forearm or the chin. This way, there is no need to connect a sensor to the scalp of the subject. As a result, the trained machine learning model provides a method for estimating sleep stages of the subject with improved comfort for the subject.

In an embodiment, the reference EMG signal comprises information about at least one physiological parameter of the reference subject during a reference sleep session. The at least one physiological parameter is different from electric activity of a muscle of the reference subject.

According to this embodiment, use is made of an insight that the EMG sensor that generates the EMG signal is not only sensitive to the electric activity of the muscle, but also to one or more other physiological parameters of the subject. For example, movement by the subject causes a change in the EMG signal generated by the EMG sensor, even if there is no change in the electric activity of the muscle near the EMG sensor. During known EMG measurements, the EMG signal is filtered to keep only the part of the EMG signal that is representative of the electric activity of the muscle and to remove the part of the EMG signal that is not representative of the electric activity of the muscle. However, the inventors have discovered that the EMG signal having both the part of the EMG signal that is representative of the electric activity of the muscle and the part of the EMG signal that is not representative of the electric activity of the muscle can be leveraged to estimate sleep stages. For example, the physiological parameter is a cardiac parameter, such as heart rate or heart rate variability or interbeat interval. The beatings of the heart of the subject cause a change in the EMG signal, because the beatings of the heart cause a vibration of the subject and the EMG sensor. For example, the physiological parameter is a respiratory parameter, such as breathing rate or depth of breath or the presence of sleep disordered breathing (SDB) events. The breathing of the subject causes a change in the EMG signal, because the breathing causes expansion and compression of the chest of the subject. The machine learning model makes use of one or more of these changes in the EMG signal to estimate the sleep stages.

According to a second aspect of the invention, there is provided a computer-implemented method for estimating sleep stages of a subject during a sleep session. The method comprises receiving an electromyography (EMG) signal representative of electric activity of a muscle of the subject during the sleep session. The method comprises providing only the EMG signal as an input to a machine learning model. The machine learning model is trained according to the first aspect of the invention. The method comprises estimating a sleep stage during the sleep session based on an output of the machine learning model.

According to the second aspect, the inventors have discovered that the EMG signal has sufficient information that allows for the estimation of sleep stages of the subject. By using the machine learning model as trained according to the first aspect on the reference EMG signal and the reference sleep stage signals, the machine learning model can be used to estimate sleep stages of a subject based on only the EMG signal. No further signals than the EMG signal are needed to estimate the sleep stages. As a result, the subject only needs to be connected to an EMG sensor, such as an EMG electrode pair. As no further signals are needed, there is no need for any other sensor to be connected to the subject. Also, the EMG sensor can be connected to the subject at a convenient location, such as on the forearm or the chin. This way, there is no need to connect a sensor to the scalp of the subject. As a result, there is provided a method for estimating sleep stages of the subject with improved comfort for the subject.

In an embodiment, the EMG signal comprises information about at least one physiological parameter of the subject during the sleep session. The at least one physiological parameter is different from electric activity of a muscle of the subject.

According to this embodiment, use is made of the insight that the EMG sensor that generates the EMG signal is not only sensitive to the electric activity of the muscle, but also to one or more other physiological parameters of the subject. For example, movement by the subject causes a change in the EMG signal generated by the EMG sensor, even if there is no change in the electric activity of the muscles near the EMG sensor. During known EMG measurements, the EMG signal is filtered to keep only the part of the EMG signal that is representative of the electric activity of the muscle and to remove the part of the EMG signal that is not representative of the electric activity of the muscle. However, the inventors have discovered that the EMG signal having both the part of the EMG signal that is representative of the electric activity of the muscle and the part of the EMG signal that is not representative of the electric activity of the muscle can be leveraged to estimate sleep stages. For example, the physiological parameter is a cardiac parameter, such as heart rate or heart rate variability or interbeat interval. The beatings of the heart of the subject cause a change in the EMG signal, because the beatings of the heart cause a vibration of the subject and the EMG sensor. For example, the physiological parameter is a respiratory parameter, such as breathing rate or depth of breath or the presence of sleep disordered breathing (SDB) events. The breathing of the subject causes a change in the EMG signal, because the breathing causes expansion and compression of the chest of the subject. The machine learning model makes use of one or more of these changes in the EMG signal to estimate the sleep stages.

In an embodiment, the computer-implemented method comprises determining whether the sleep stage is a Rapid Eye Movement (REM) sleep stage. The computer-implemented method comprises determining, in case the sleep stage is a REM sleep stage, whether a REM sleep behavior disorder (RBD) is present based on the EMG signal.

Durin REM sleep, healthy people experience a reduced muscle tone for many of the body's muscles. This reduction of muscle tone is referred to as muscle atonia. During REM sleep, the most vivid dreaming occurs. The muscle atonia prevents the sleeper from physically reacting to a dream, for example by kicking, punching, or grabbing. The muscle atonia is accurately measurable via an EMG measured, because the electric activity of the muscle gives an accurate representation of whether muscle atonia is present or not. Subjects who suffer from a condition called REM sleep behavior disorder (RBD), do not experience muscle atonia during REM sleep, or do not experience enough muscle atonia during REM sleep. RBD is known to be a powerful predictor for the future development of Parkinson's disease and other neurodegenerative diseases. According to this embodiment, it is possible to use the EMG signal without the need to perform any other type of measurement on the subject, to determine whether the subject is in a REM sleep stage, and when in a REM sleep stage, whether RBD is present. When determining RBD is present, treatment may be started to treat the RBD, for example by using medication. Further, the simple and convenient way to obtain an EMG signal allows for cost-effective screening of subjects for the presence of RBD. Because of this screening, subjects that are likely to develop Parkingson's disease may be timely identified.

In an embodiment, the computer-implemented method comprises providing, in case the sleep stage is a REM sleep stage, a portion of the EMG signal corresponding to the sleep stage as an input to an RBD classifier. The RBD classifier is configured to classify the subject to a first class or a second class based on the portion of the EMG signal. The first class represents the RBD is present. The second class represents the RBD is not present.

According to this embodiment, a portion of the EMG signal corresponding to the REM sleep stage is input in the RBD classifier. For a subject without RBD, that portion of the EMG signal shows muscle atonia. However, for a subject suffering from RBD, that portion of the EMG signal does not show muscle atonia or does not show sufficient muscle atonia. The RBD classifier is configured to classify the subject to either the first class or to the second class. In case the portion of the EMG signal does not show muscle atonia or does not show sufficient muscle atonia, the RBD classifier classifies the subject to the first class. In case the portion of the EMG signal shows muscle atonia, the RBD classifier classifies the subject to the second class.

For example, the first class and/or the second class comprises multiple subclasses. The subclasses are, for example, representative of a probability of the presence of RBD. For example, the first class has three subclasses: one subclass with a high probability that the RBD is present, one subclass with a medium probability that the RBD is present, and one subclass with a low probability that the RBD is present. For example, the second class has three subclasses: one subclass with a high probability that the RBD is not present, one subclass with a medium probability that the RBD is not present, and one subclass with a low probability that the RBD is not present. For example, in case the RBD classifier classifies the subject to a subclass with a low probability, an output signal is generated to suggest an action to further investigate whether the RBD is present.

In an embodiment, the computer-implemented method comprises deriving, with the RBD classifier, from the EMG signal, an RBD feature representative of the presence of the RBD. The computer-implemented comprises performing a comparison between the RBD feature and a RBD reference feature. The computer-implemented comprises classifying, with the RBD classifier, the subject to the first class or the second class based on the comparison.

According to this embodiment, the RBD classifier is used to derive from the EMG signal, the RBD feature. For example, the RBD feature is a measure of the EMG signal energy, or the EMG signal power, or a wavelet decomposition of the EMG signal, or a power spectral density of the EMG signal. For example, the RBD feature represents the muscle atonia or the lack of muscle atonia or an amount of muscle atonia. For example, the RBD feature represents an intensity level of the electric activity of the muscle or a frequency of the electric activity of the muscle or a pattern of the electric activity of the muscle over time. The RBD classifier compares the RBD feature with the RBD reference feature. For example, the RBD reference feature is the same feature as the RBD feature. For example, the RBD reference feature represents the RBD feature for a reference subject having RBD. For example, the RBD reference feature represents the RBD feature for a reference subject not having RBD. For example, the RBD reference feature represents both the RBD feature for a reference subject having RBD and the RBD feature for a reference subject not having RBD. For example, the RBD classifier determines a difference between the RBD feature and the RBD reference feature. For example, RBD classifier determines whether the difference exceeds a threshold.

In an embodiment, the computer-implemented method comprises determining, with the RBD classifier, an amount of muscle tone of the subject based on the portion of the EMG signal. The computer-implemented method comprises performing a comparison between the amount of muscle tone and a reference muscle tone. The computer-implemented method comprises classifying, with the RBD classifier, the subject to the first class or the second class based on the comparison.

According to this embodiment, the RBD classifier determines the amount of muscle tone based on the portion of the EMG signal. The amount of muscle tone is an accurate measure for the presence of muscle atonia. In case of the subject has insufficient muscle atonia because of the RBD, the amount of muscle tone is higher than in case of sufficient muscle atonia.

In an embodiment, the EMG signal is a single-lead EMG signal.

According to this embodiment, a single-lead EMG signal is used. To obtain the single-lead EMG signal, only a single EMG sensor is required. This way, the EMG signal is obtained with only a minimum discomfort to the patient.

In an embodiment, the EMG signal comprises an unfiltered signal generated by at least one electrode pair.

According to this embodiment, the unfiltered signal generated by the at least one electrode pair comprises information that is representative of the electric activity of the muscles and information that is not representative of the electric activity of the muscle. However, the inventors have discovered that the EMG signal that has a part representative of the electric activity of the muscle, and that has a part that is not representative of the electric activity of the muscle can be leveraged to estimate sleep stages.

In an embodiment, the EMG signal is representative of electric activity of a muscle of a chin, a leg, a neck, an arm, or a jaw of the subject.

According to this embodiment, the EMG signal is generated by an EMG sensor that is located on a chin, a leg, a neck, an arm, or a jaw of the subject. These locations provide for an easy application of the EMG sensor without much discomfort to the subject. Further, these locations provide for an accurate EMG signal, as the EMG signal is not significantly disturbed by other electrical signals, such as electrical signals from the brain or electrical signals from the heart. However, some disturbance of these other electrical signals may be leveraged by the trained machine learning model to determine the sleep stages.

In an embodiment, the EMG signal is representative of electric activity of a muscle of a limb of the subject. The computer-implemented method comprises estimating a movement of the limb based on the EMG signal. The computer-implemented method comprises determining a presence of periodic limb movement disorder (PLMD) based on the movement and the sleep stage.

According to this embodiment, the EMG signal is not only used to estimate the sleep stage, but also whether there is movement of the limb of the subject. PLMD is a sleep disorder in which a subject periodically moves limbs, most noticeably the legs, while the subject is asleep. The sleep stage is taken into account, because limb movement while the subject is awake is not indicative of PLMD. The sleep stage is taken into account because due to the muscle atonia during REM, the subject does not have periodic limb movement during REM even if suffering from PLMD.

According to a third aspect of the invention, there is provided a processing system configured to perform the computer-implemented method of the second aspect.

According to a fourth aspect of the invention, there is provided a computer program product, comprising instructions which, when executed by a processing system, cause the processing system to carry out the computer-implemented method of the second aspect.

According to a fifth aspect of the invention, there is provided a computer-readable storage medium comprising instructions which, when executed by a processing system, cause the processing system to carry out the method of the second aspect.

According to a sixth aspect of the invention, there is provided an electromyography (EMG) device adapted to measure electric activity of a muscle of a subject during a sleep session. The EMG device comprises the processing system according to the third aspect of the invention, and a sensor interface. The sensor interface is adapted to couple to at least one sensor adapted to generate an EMG signal representative of electric activity of a muscle of the subject.

In an embodiment, the EMG device comprises an output interface adapted to generate an output signal representative of the sleep stage.

According to this embodiment, a professional caregiver is able to use the output signal as a part of a diagnosis or when treating the subject.

In an embodiment, the output interface is adapted to generate a further output signal representative of a presence of the RBD.

According to this embodiment, a professional caregiver is able take actions in case the RBD is presence, for example, to prevent or to delay the development of Parkingson's disease.

### BRIEF DESCRIPTION OF THE DRAWINGS

Exemplary embodiments will now be described, by way of example only, with reference to the following Figures, in which:
FIG. 1 depicts an EMG device according to a first embodiment of the invention;
FIG. 2 depicts training of a machine learning model according to a second embodiment of the invention;
FIG. 3 depicts a computer-implemented method for estimating sleep stages of a subject during a sleep session according to a third embodiment of the invention;
FIG. 4 depicts a computer-implemented method according to a fourth embodiment of the invention;
FIG. 5 depicts a processing system according to a fifth embodiment of the invention;
FIG. 6 depicts a computer-implemented method according to a sixth embodiment of the invention;
FIG. 7 depicts a power level in [dB] of the EMG signal during a REM sleep stage.

### DETAILED DESCRIPTION OF EMBODIMENTS

It should be understood that the detailed description and specific examples, while indicating exemplary embodiments of the devices and methods, are intended for purposes of illustration only and are not intended to limit the scope of the invention. These and other features, aspects, and advantages of the devices and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

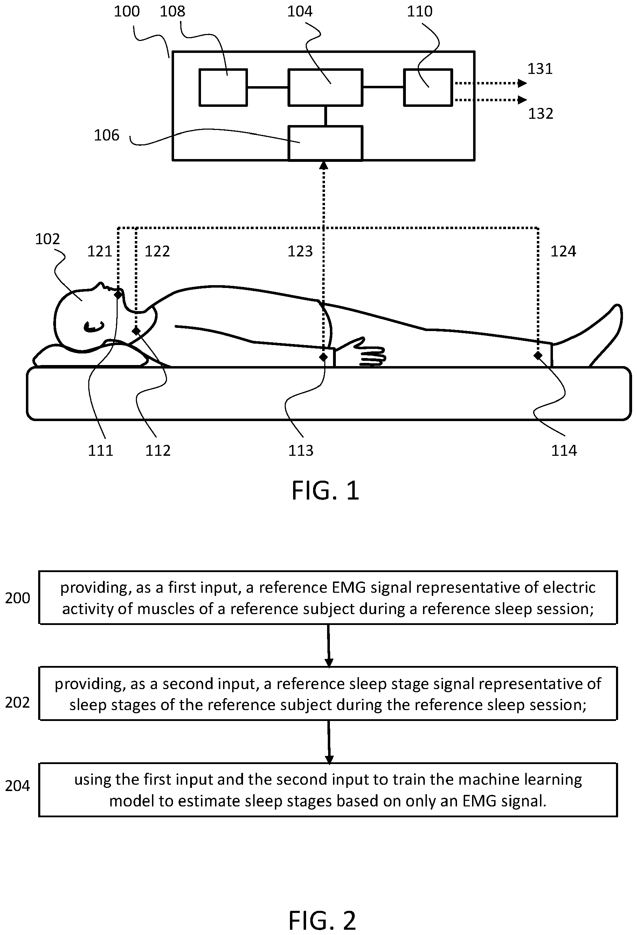

FIG. 1 depicts an electromyography (EMG) device 100 according to a first embodiment of the invention. The EMG device 100 is adapted to measure electric activity of a muscle of a subject 102 during a sleep session. The EMG device 100 comprises a processing system 104, a sensor interface 106, a memory 108, and an output interface 110. The processing system 104 is configured to perform a computer-implemented method as described later on. The sensor interface 106 is adapted to couple to at least one EMG sensor 111-114. The at least one EMG sensor 111-114 is adapted to generate an EMG signal 121-124 representative of electric activity of a muscle of the subject 102.

FIG. 1 depicts the four EMG sensors 111-114 attached to the subject 102. EMG sensor 111 is adapted to generate the EMG signal 121 representative of electric activity of a muscle of the chin of the subject 102 or the jaw of the subject 102, or both the chin and the jaw of the subject 102. EMG sensor 112 is adapted to generate the EMG signal 122 representative of electric activity of a muscle of the neck of the subject 102. EMG sensor 113 is adapted to generate the EMG signal 123 representative of electric activity of a muscle of the arm of the subject 102. EMG sensor 114 is adapted to generate the EMG signal 124 representative of electric activity of a muscle of the leg of the subject 102. In an embodiment, only a subset of the EMG sensors and EMG signals is used, for example, only one of the EMG sensors 111-114 and only one of the EMG signals 121-124 is used. For example, one of the EMG signals 121-124 is a single-lead EMG signal, or a plurality of the EMG signals 121-124 are single-lead EMG signals. For example, the EMG signal 121-124 comprises an unfiltered signal generated by at least one electrode pair.

The EMG sensors 111-114 comprise the at least one electrode pair. For example, the two electrodes of the electrode pair are arranged close together, for example at a distance of less than 10 cm or less than 5 cm or less than 2 cm or less than 1 cm from each other. The electrode pair is arranged to detect electric activity of a single muscle or of multiple muscles near the electrode pair. For example, the electrode pair is arranged to detect a change in voltage of a single muscle or of multiple muscles near the electrode pair. For example, one or more of EMG sensors 111-114 comprises three electrodes each. The three electrodes are arranged close together, for example at a distance of less than 10 cm or less than 5 cm or less than 2 cm or less than 1 cm from each other.

The processing system 104 is configured to receive the at least one EMG signal 121-124 from the sensor interface 106.

The processing system 104 makes use of a machine learning model. FIG. 2 shows the training of the machine learning model according to a second embodiment of the invention.

The computer-implemented method for training the machine learning model comprises providing, 200, as a first input, a reference electromyography (EMG) signal representative of electric activity of a muscle of a reference subject during a reference sleep session. For example, the reference subject is a healthy subject or a subject having a condition. For example, the reference subject is a different subject than the subject. In another example, the reference subject is the same subject as the subject 102. In this example, the machine learning model is specifically trained to the subject 102. For example, multiple reference EMG signals from multiple reference subjects are provided as the first input. For example, multiple reference EMG signals from multiple reference sleep sessions are provided as the first input. For example, multiple reference EMG signals from multiple reference sleep sessions of multiple reference subjects are provided as the first input. The reference sleep session is, for example, a sleep session at a sleep center or hospital, or at the reference subject's home. For example, the reference EMG signal is generated during only a part of a reference sleep session. Preferably, the reference EMG signal is generated by a sensor that is on a same location on the reference subject as the sensor that generates the EMG signal is on the subject 102. For example, both the reference EMG signal and the EMG signal 111-114 are generated by sensors arranged on the chin.

The computer-implemented method for training the machine learning model comprises providing, 202, as a second input, a reference sleep stage signal representative of sleep stages of the reference subject 102 during the reference sleep session.

For example, the reference sleep stage signal is generated based on polysomnography (PSG). For example, one or more signals from the PSG are manually annotated by a sleep technician to determine the sleep stage of the reference sleep stage signal.

For example, the reference sleep stage signal is generated based on electroencephalography (EEG) and/or electrooculography (EOG). For example, the reference sleep stage signal is generated based on respiratory information. For example, the reference sleep stage signal is generated based on cardiac information, such as heart rate, or heart rate variability, or interbeat-intervals. For example, an automatic sleep stage scoring algorithm, such as Somnolyzer^{™} by Philips, is used to generate the reference sleep stage signal based on one or more physiological parameters of the reference subject. For example, the physiological parameter comprises a cardiac parameter, or a respiratory parameter, or a brain activity parameter.

The computer-implemented method for training the machine learning model comprises using, 204, the first input and the second input to train the machine learning model to estimate sleep stages based on only an EMG signal. The second input provides an accurate estimation of the reference sleep stages during the reference sleep session. Based on the second input, the machine learning model is trained to estimate sleep stages based on only an EMG signal. The inventors have discovered that an EMG signal has sufficient information to allow the machine learning model to be trained to estimate the sleep stages based on only an EMG signal.

For example, the sleep stages are classified into two sleep stage classes, i.e., a wake sleep stage class and a non-wake sleep stage class. During the wake sleep stage, the subject 102 is awake. During the non-wake sleep stage, the subject 102 is asleep. In another example, the sleep stages are classified into two sleep stage classes, i.e., a REM sleep stage class and a non-REM sleep stage class. Using these two sleep stage classes is useful for determining the presence of RBD. During the REM sleep stage, the subject 102 has Rapid Eye Movement during sleep. During the non-REM sleep stage, the subject 102 has no Rapid Eye Movement. For example, the sleep stages are classified into three classes. The three classes are a wake sleep stage class, a REM sleep stage class, and a non-REM sleep stage class. During the REM sleep stage class, the subject 102 is asleep and has Rapid Eye Movements, whereas during the non-REM sleep stage class, the subject 102 is asleep without Rapid Eye Movements. For example, the sleep stages are classified into four classes. The four classes are a wake sleep stage class, a REM sleep stage class, a light sleep stage class, and a deep sleep stage class. For example, the sleep stages are classified into the classes N1, N2, N3, REM and Wake.

For example, the reference EMG signal comprises information about at least one physiological parameter of the reference subject during a reference sleep session. The at least one physiological parameter is different from electric activity of a muscle of the reference subject. Although the EMG sensor is adapted to generate the EMG signal based on only electric activity of a muscle near the EMG sensor, the EMG signal is affected by other physiological parameters as well. For example, the EMG signal is affected by a cardiac parameter, such as heart rate or heart rate variability or interbeat-intervals. For example, the EMG signal is affected by a respiratory parameter, such as breathing rate or depth of breath or the presence of sleep disordered breathing (SDB) events. By training the machine learning model based on the EMG signal, the machine learning model is able to leverage the effects of the other physiological parameters on the EMG signal to estimate the sleep stages.

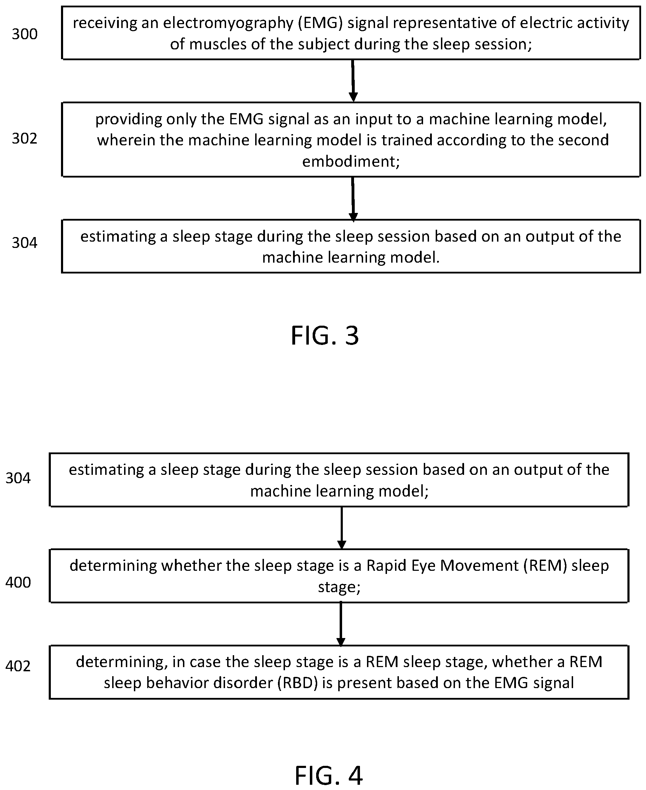

FIG. 3 depicts a computer-implemented method for estimating sleep stages of a subject 102 during a sleep session according to a third embodiment of the invention. For example, the computer-implemented method according to the third embodiment is used in the EMG device 100 according to the first embodiment.

The computer-implemented method according to the third embodiment comprises, 300, receiving an electromyography (EMG) signal representative of electric activity of a muscle of the subject 102 during the sleep session. The computer-implemented method comprises, 302, providing only the EMG signal as an input to a machine learning model. The machine learning model is trained according to the second embodiment. The computer-implemented method comprises, 304, estimating a sleep stage during the sleep session based on an output of the machine learning model.

The computer-implemented method makes use of the trained machine learning model of the second embodiment, by providing only the EMG signal as an input to the machine learning model. The machine learning model is trained to estimate the sleep stages based on the EMG signal.

For example, the EMG signal comprises information about at least one physiological parameter of the subject 102 during the sleep session. The at least one physiological parameter is different from electric activity of a muscle of the subject 102.

FIG. 4 depicts a computer-implemented method according to a fourth embodiment of the invention. The fourth embodiment has, for example, the same elements as the third embodiment. For example, the computer-implemented method according to the fourth embodiment is used in the EMG device 100 according to the first embodiment.

In the fourth embodiment, the computer-implemented method comprises, 400, determining whether the sleep stage is a Rapid Eye Movement (REM) sleep stage. The computer-implemented method comprises, 402, determining, in case the sleep stage is a REM sleep stage, whether a REM sleep behavior disorder (RBD) is present based on the EMG signal.

Based on estimating, 304, the sleep stage during the sleep session based on the output of the machine learning model, the method is able to determine whether the sleep stage is a REM sleep stage or not a REM sleep stage. During a REM sleep stage, a subject 102 has rapid eye movements. In case the sleep stage is a REM sleep stage, the method uses the EMG signal to determine whether a sleep behavior disorder (RBD) is present.

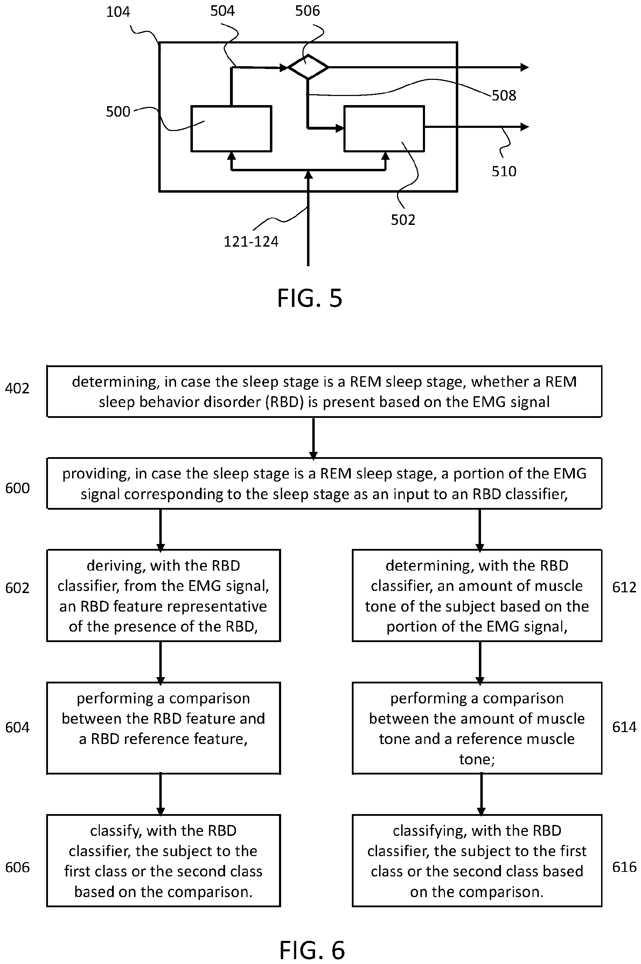

FIG. 5 depicts a processing system 104 according to a fifth embodiment of the invention. The fifth embodiment has, for example, the same elements as the fourth embodiment. For example, the computer-implemented method according to the fifth embodiment is used in the EMG device 100 according to the first embodiment.

In the fifth embodiment, processing system 104 comprises a machine learning model 500 and an RBD classifier 502. The machine learning model 500 is configured to estimate sleep stages based on the EMG signal 121-124. The machine learning model 500 is, for example, the same machine learning model as in the third embodiment. For example, the machine learning model 500 is trained according to the second embodiment.

The machine learning model 500 outputs a sleep stage output signal 504 representative of one or more sleep stages. At 506, the processing system 104 determines whether one or more of the sleep stages is a REM sleep stage. The sleep stage output signal 504 is output from the processing system 104. For example, the EMG device 100 comprises an output interface 110. The output interface 110 is adapted to generate an output signal 131 representative of the sleep stage. The processing system 104 provides the sleep stage output signal 504 to the output interface 110.

In case the sleep stage is a REM sleep stage, the EMG signal corresponding to the sleep stage is provided as an input to an RBD classifier 502. The RBD classifier 502 is configured to classify the subject 102 to a first class or a second class based on the EMG signal. The first class represents the RBD is present. The second class represents the RBD is not present. The classification of the subject 102 to either the first class or the second class is output from the processing system 104 via an RBD output signal 510. For example, the EMG device 100 comprises the output interface 110. The output interface 110 is adapted to generate a further output signal 132 representative of a presence of the RBD. The processing system 104 provides the RBD output signal 510 to the output interface 110.

FIG. 6 depicts a computer-implemented method according to a sixth embodiment of the invention. For example, the method according to the sixth embodiment is performed by the processing system 104 according to the fifth embodiment. The sixth embodiment has, for example, the same elements as the fourth embodiment. For example, the computer-implemented method according to the sixth embodiment is used in the EMG device 100 according to the first embodiment.

In the sixth embodiment, the computer-implemented method comprises, 402, determining, in case the sleep stage is a REM sleep stage, whether a REM sleep behavior disorder (RBD) is present based on the EMG signal 121-124. It is determined whether the RBD is present by providing, 600, in case the sleep stage is a REM sleep stage, a portion of the EMG signal 121-124 corresponding to the sleep stage as an input to the RBD classifier 502. The RBD classifier 502 is configured to classify the subject 102 to a first class or a second class based on the portion of the EMG signal 121-124. The first class represents the RBD is present. The second class represents the RBD is not present.

For example, the computer-implemented method comprises deriving, 602, with the RBD classifier 502, from the EMG signal 121-124, an RBD feature representative of the presence of the RBD. The method comprises, 604, performing a comparison between the RBD feature and an RBD reference feature. The method comprises classifying, 606, with the RBD classifier 502, the subject 102 to the first class or the second class based on the comparison.

For example, in addition or alternative to the steps 602-604, the computer-implemented method comprises determining, 612, with the RBD classifier 502, an amount of muscle tone of the subject 102 based on the portion of the EMG signal 121-124. The method comprises performing, 614, a comparison between the amount of muscle tone and a reference muscle tone. The method comprises classifying, 616, with the RBD classifier 502, the subject 102 to the first class or the second class based on the comparison.

For example, a computer program product is provided. The computer program product comprises instructions which, when executed by the processing system 104, cause the processing system 104 to carry out the computer-implemented method according to the third embodiment, the fourth embodiment or the sixth embodiment.

For example, a computer-readable storage medium is provided. The computer-readable storage medium comprises instructions which, when executed by the processing system 104, cause the processing system 104 to carry out the method according to the third embodiment, the fourth embodiment or the sixth embodiment. For example, the computer-readable storage medium comprises the memory 108.

A proof-of-concept of the invention is provided as follows. A prototype was developed based on real single-channel EMG data. To that end, 1851 recordings from the SOMNIA database, and 97 recordings from the HealthBed database were used. These records were split into 1347 train recordings, 100 validation recordings, and 500 test recordings. A deep neural network was trained to perform sleep staging on this task. The deep neural network is an example of the machine learning model 500. Table 1 shows the sleep staging performance for different numbers of sleep stages classes. In one case, 5 sleep stage classes were used, i.e., Wake, N1, N2, N3, and REM. In another case, 4 sleep stage classes were used, i.e., Wake, N1/N2, N3, and REM. In this case, N1 and N2 were merged in a single class. In yet another case, 3 sleep stage classes were used, i.e., Wake, Non-REM, and REM. In this case, all Non-REM classes were merged. It is noted that these 3 sleep stage classes are sufficient for RBD detection.

The following tables show the accuracy for the three different cases, i.e., with different number of sleep stage classes. Tabel 1 shows the average accuracy for healthy subjects and subjects with a diverse range of sleep disorders. Table 2 shows the accuracy divided for subjects that are healthy, have sleep disorders without RBD, and have RBD.

The column "EMG signal" represents at which location the EMG sensor is attached to the subjects. The expressions 'Chin1', `Chin2', and 'Chin3' refer to the locations of EMG electrodes on the chin according to the guidelines of the American Academy of Sleep Medicine.

**Table 1: Average sleep stage classification accuracy for 3, 4 or 5 sleep stage classes.**

| **EMG signal** | **5 class accuracy** | **4 class accuracy** | **3 class accuracy** |
|---|---|---|---|
| Chin1-Chin2 | 74.9 | 80.9 | 88.1 |
| Chin2-Chin3 | 74.5 | 80.6 | 87.8 |
| Chin1-Chin3 | 74.9 | 80.9 | 88.0 |
| Forearm Left | 63.7 | 69.5 | 78.8 |
| Forearm Right | 63.2 | 69.1 | 78.2 |
| Leg Left | 66.9 | 72.2 | 81.2 |
| Leg Right | 66.7 | 72.0 | 80.8 |
| Neck Left | 66.2 | 72.4 | 80.5 |
| Neck Right | 67.0 | 73.1 | 81.7 |

**Table 2: Average accuracy for different groups of subjects using 3 sleep stage classes.**

| **EMG signal** | **3 class accuracy for healthy** | **3 class accuracy for disordered without RBD** | **3 class accuracy for RBD** |
|---|---|---|---|
| Chin1-Chin2 | 87.5 | 88.3 | 85.3 |
| Chin2-Chin3 | 89.0 | 87.9 | 84.7 |
| Chin1-Chin3 | 87.8 | 88.2 | 85.0 |
| Forearm Left | - | 77.7 | 80.6 |
| Forearm Right | - | 77.2 | 79.9 |
| Leg Left | 84.0 | 81.0 | 80.4 |
| Leg Right | 82.8 | 80.7 | 79.5 |
| Neck Left | - | 80.5 | - |
| Neck Right | - | 81.7 | - |

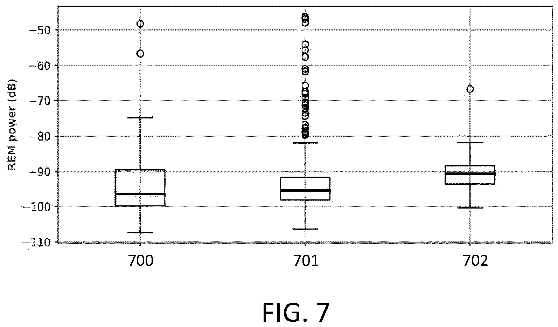

Another proof-of-concept of the invention is provided as follows. As EMG sensors, the 'chin1-chin3' were used according to the AASM guidelines. With the EMG signals from these EMG sensors, periods during a sleep session with REM sleep were detected. The average power of the EMG signals during these periods was calculated. This measurement was conducted on multiple subjects. The outcome of the measurement is depicted in FIG. 7. FIG. 7 depicts on the y-axis the power level in [dB] of the EMG signal during a REM sleep stage. Three boxplots are show respectively for healthy subjects at 700, subjects with sleep disorders other than RBD at 701, and subjects with RBD at 702. From the boxplots it becomes clear that for subjects without RBD, the power level is substantially lower than for subjects with RBD. The average power level for subjects without RBD is around between -95 and -97 dB, whereas the average power level for subjects with RBD is around -90 dB. This way, FIG. 7 shows that the power of the EMG signal during REM sleep can be used for determining the presence of RBD.

In addition or alternative to the power of the EMG signal, more sophisticated features of the EMG signal can be used to further improve RBD detection performance. These more sophisticated features may allow a further distinction between generally elevated muscle tone during RBD versus the periodic (phasic) and naturally occurring short bursts in EMG activity during REM in non-RBD subjects.

It will be understood that the disclosed methods are computer-implemented methods. As such, there is also proposed a concept of a computer program comprising code means for implementing any described method when said program is run on a processing system 104.

The skilled person would be readily capable of developing a processing system 104 for carrying out any herein described method. Thus, each step of a flow chart may represent a different action performed by a processing system 104, and may be performed by a respective module of the processing system 104.

The processing system 104 can be implemented in numerous ways, with software and/or hardware, to perform the various functions required. The processing system 104 employs, for example, one or more microprocessors that may be programmed using software (e.g. microcode) to perform the required functions. The processing system 104 may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processing system 104 may be embodied as a digital and/or analog processing system 104.

In various implementations, the processing system 104 may be associated with one or more storage media such as volatile and non-volatile computer memory 108 such as RAM, PROM, EPROM, and EEPROM. The storage media may be encoded with one or more programs that, when executed on one or more processing system 104s and/or controllers, perform the required functions. Various storage media may be fixed within a processing system 104 or controller may be transportable, such that the one or more programs stored thereon can be loaded into a processing system 104.

Functions implemented by a processor may be implemented by a single processor or by multiple separate processing units which may together be considered to constitute a "processor". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A computer-implemented method for training a machine learning model (500), the method comprising:
providing (200), as a first input, a reference electromyography (EMG) signal representative of electric activity of a muscle of a reference subject during a reference sleep session;
providing (202), as a second input, a reference sleep stage signal representative of sleep stages of the reference subject during the reference sleep session;
using (204) the first input and the second input to train the machine learning model (500) to estimate sleep stages based on only an EMG signal (121-124).

2. The computer-implemented method according to claim 1,
wherein the reference EMG signal comprises information about at least one physiological parameter of the reference subject during a reference sleep session,
wherein the at least one physiological parameter is different from electric activity of a muscle of the reference subject.

3. A computer-implemented method for estimating sleep stages of a subject (102) during a sleep session, the method comprising:
receiving (300) an electromyography (EMG) signal (121-124) representative of electric activity of a muscle of the subject (102) during the sleep session;
providing (302) only the EMG signal (121-124) as an input to a machine learning model (500),
wherein the machine learning model (500) is trained according to claim 1 or 2;
estimating (304) a sleep stage during the sleep session based on an output of the machine learning model (500).

4. The computer-implemented method according to claim 3,
wherein the EMG signal (121-124) comprises information about at least one physiological parameter of the subject (102) during the sleep session,
wherein the at least one physiological parameter is different from electric activity of a muscle of the subject (102).

5. The computer-implemented method according to claim 3 or 4, comprising:
determining (400) whether the sleep stage is a Rapid Eye Movement (REM) sleep stage;
determining (402), in case the sleep stage is a REM sleep stage, whether a REM sleep behavior disorder (RBD) is present based on the EMG signal (121-124).

6. The computer-implemented method according to claim 5, comprising
providing (600), in case the sleep stage is a REM sleep stage, a portion of the EMG signal (121-124) corresponding to the sleep stage as an input to an RBD classifier (502),
wherein the RBD classifier (502) is configured to classify the subject (102) to a first class or a second class based on the portion of the EMG signal (121-124),
wherein the first class represents the RBD is present,
wherein the second class represents the RBD is not present.

7. The computer-implemented method according to claim 6, comprising:
deriving (602), with the RBD classifier (502), from the EMG signal (121-124), an RBD feature representative of the presence of the RBD,
performing (604) a comparison between the RBD feature and a RBD reference feature,
classifying (606), with the RBD classifier (502), the subject (102) to the first class or the second class based on the comparison.

8. The computer-implemented method according to claim 6 or 7, comprising
determining (612), with the RBD classifier (502), an amount of muscle tone of the subject (102) based on the portion of the EMG signal,
performing (614) a comparison between the amount of muscle tone and a reference muscle tone;
classifying (616), with the RBD classifier (502), the subject (102) to the first class or the second class based on the comparison.

9. The computer-implemented method according to any one of claims 3-8, wherein the EMG signal (121-124) is a single-lead EMG signal.

10. The computer-implemented method according to any one of claims 3-9, wherein the EMG signal (121-124) comprises an unfiltered signal generated by at least one electrode pair.

11. The computer-implemented method according to any one of claims 3-10, wherein the EMG signal (121-124) is representative of electric activity of a muscle of a chin, a leg, a neck, an arm, or a jaw of the subject (102).

12. A processing system (104) configured to perform the computer-implemented method of any of claims 3-11.

13. A computer program product, comprising instructions which, when executed by a processing system (104), cause the processing system (104) to carry out the computer-implemented method of claims 3-11.

14. An electromyography (EMG) device (100) adapted to measure electric activity of a muscle of a subject (102) during a sleep session, the EMG device (100) comprising;
the processing system (104) according to claim 12;
a sensor interface (106) adapted to couple to at least one sensor (111-114) adapted to generate an EMG signal (121-124) representative of electric activity of a muscle of the subject (102).

15. The EMG device (100) according to claim 14, comprising an output interface (110) adapted to generate an output signal (131) representative of the sleep stage.
